# EUROPÄISCHE PATENTANMELDUNG

(11) **EP 1 510 536 A1**
(43) Veröffentlichungstag der Anmeldung: **02.03.2005**
(21) Anmeldenummer: 04016868.4
(22) Anmeldetag: 16.07.2004
(51) Int. Cl.: C08G 65/30, C07C 41/34

(54) **Verfahren zur Herstellung von Polyetheralkoholen**

(30) Priorität: 21.08.2003 DE 10338827
(71) Anmelder: BASF AKTIENGESELLSCHAFT, 67056 Ludwigshafen (DE)
(72) Erfinder: Groer, Peter, Dr., 65812 Bad Soden (DE); Ostrowski, Thomas, Dr., 68167 Mannheim (DE); Buss, Christian, Dr., 01129 Dresden (DE)

(57) **Zusammenfassung**

Gegenstand der Erfindung ist ein Verfahren zur Herstellung von Polyetheralkoholen durch Anlagerung von Alkylenoxiden an H-funktionelle Startsubstanzen, dadurch gekennzeichnet, dass die Startsubstanzen ausgewählt sind aus der Gruppe, enthaltend Alkohole mit mindestens zwei Hydroxylgruppen im Molekül und/oder Amine mit mindestens einer Aminogruppe im Molekül und einem Molekulargewicht von maximal 200 g/mol, und dass die Umsetzung in Anwesenheit von mindestens einer reduzierend wirkenden, nicht flüchtigen anorganischen Verbindung durchgeführt und/oder der Polyetheralkohol nach der Umsetzung mit den Alkylenoxiden einer Behandlung mit mindestens einer reduzierend wirkenden, nicht flüchtigen anorganischen Verbindung unterzogen wird.

## Beschreibung

Polyetheralkohole, ihre Herstellung sowie ihre Verwendung sind lange bekannt und vielfach in der Literatur beschrieben. Die Herstellung erfolgt üblicherweise durch Polymerisation von niederen Alkylenoxiden, zumeist in Anwesenheit von H-funktionellen Startsubstanzen und Katalysatoren. Der Einsatz der Polyetheralkohole erfolgt überwiegend zur Herstellung von Polyurethanen durch Umsetzung mit Isocyanaten.

Bei der Polymerisation der Alkylenoxide sowie der Lagerung der Polyetheralkohole laufen häufig Nebenreaktionen ab, die zur Bildung von flüchtigen Verbindungen führen. Diese flüchtigen Verbindungen besitzen einen starken Eigengeruch, der sich auch auf die Polyurethane überträgt. Dies wird häufig als Qualitätsmangel empfunden. So fordern viele Kunden, insbesondere auf dem Gebiet der Weichschaumstoffe und bei Automobilanwendungen, sehr geringe Werte für flüchtige Verbindungen, insbesondere für Aldehyde.

Weiterhin ist für einige Anwendungen von Polyetheralkoholen eine niedrige Säurezahl wichtig. Wird die Säurezahl durch organische Säuren hervorgerufen, kann diese Säure nicht einfach neutralisiert und entfernt werden, beispielsweise durch Filtration, da derartige Säuregruppen Bestandteil der Polyetherkette sein können. Eine Neutralisation führt zwar zur Abnahme der Säurezahl, es kommt jedoch zu einem Ansteigen der Kaliumwerte, was ebenfalls häufig unerwünscht ist.

Die beschriebenen Säuren und die flüchtigen Verbindungen lassen sich zumeist auf einen oxidativen Abbau der Polyether zurückführen.

Diese Abbauprozesse werden durch geringe Mengen an Sauerstoff, die nicht vollständig auszuschließen sind, initiiert und laufen unter den Bedingungen der Synthese, wie erhöhte Temperaturen und stark alkalisches Medium, viel schneller ab als bei Raumtemperatur. Die bei dieser Reaktion gebildete Peroxide werden bei der nachfolgenden Aufarbeitung nur teilweise zerstört und können als maskierte Aldehyde oder Säuren erst später zu einer Produktverschlechterung führen. Um das Geruchsproblem der Polyetheralkohole nachhaltig zu lösen, sollte die Bildung von Nebenprodukten, die zu einem Geruch der Polyetheralkohole führen, bereits bei der Synthese unterbunden werden.

Es ist bekannt, die Umsetzung von bestimmten Ausgangsverbindungen, die ihrerseits einen Eigengeruch aufweisen, mit Alkylenoxiden in Anwesenheit von reduzierenden Verbindungen durchzuführen. Dabei handelt es sich jedoch um Alkoxylate mit sehr speziellen und für den Einsatz in Polyurethanen unüblichen Startsubstanzen.

In DE 42 42 017 wird ein Verfahren zur Herstellung von Tensiden durch Umsetzung von Fettsäureestern mit Ethylenoxid und Propylenoxid beschrieben, das in Anwesenheit von Reduktionsmitteln durchgeführt wird. Durch die Anwesenheit der Reduktionsmittel soll die Farbzahl der Produkte verbessert werden. Außerdem weisen die so hergestellten Produkte einen geringeren Gehalt an Formaldehyd und freier Ameisensäure auf. Besonders vorteilhaft kann dieses Verfahren bei Einsatz von niedrig veredelten Produkten, wie Tri- oder Partialglyzeriden, eingesetzt werden, da bei derartigen Ausgangsstoffen Produkte mit besonders schlechte Farbzahlen erhalten werden. Damit können aufwendige Verfahren zur Aufhellung der Produkte vermieden werden. In diesem Verfahren geht es vorrangig darum, Probleme, die durch die verwendeten Startsubstanzen auftreten, zu minimieren.

In WO 99/16811 wird ein Verfahren zur Herstellung von ethxylierten Polyaminen beschrieben, die insbesondere als Zusatzstoffe für die Textilveredlung eingesetzt werden können. Zur Senkung des Aldehydgehaltes, der für den unerwünschten Geruch der Verbindungen verantwortlich ist, werden in diesem Dokument eine Reihe von Maßnahmen vorgeschlagen. So ist es beispielsweise möglich, das Reaktionsprodukt nach der Umsetzung mit Natriumsulfit zu behandeln. Eine weitere Möglichkeit ist die Zugabe von Salzen des Borhydrids zu der Reaktionsmischung vor oder während der Anlagerung des Ethylenoxids. Der Geruch kommt bei diesem Verfahren hauptsächlich durch nicht umgesetzten Ethylenoxid oder durch Quarternisierung von tertiären Aminogruppen, die in den als Startsubstanz verwendeten Polyaminen vorhanden sind, zustande.

Die in den beiden genannten Dokumenten beschriebenen alkoxylierten Verbindungen unterscheiden sich, wie erwähnt, in ihrem Aufbau deutlich von den Polyetheralkoholen, die zur Herstellung von Polyurethanen eingesetzt werden.

Aufgabe der Erfindung war es, eine einfache Möglichkeit zur Herstellung von Polyetheralkoholen, die ihrerseits zur Herstellung Polyurethanen eingesetzt werden können, zu finden, die einen sehr geringen Gehalt an flüchtigen Bestandteilen enthalten und daher keinen Geruch aufweisen. Die Reduzierung des Gehaltes an flüchtigen Bestandteilen sollte kostengünstig und mit der üblichen Technologie und den üblichen Einsatzstoffen bei der Herstellung von Polyetheralkoholen kompatibel sein. Außerdem sollten die so hergestellten Polyetheralkohole eine niedrige Säurezahl aufweisen.

Die Aufgabe konnte überraschenderweise gelöst werden, indem die Anlagerung der Alkylenoxide in Anwesenheit von Verbindungen, die reduzierend wirken, durchgeführt wird.

Gegenstand der Erfindung ist ein Verfahren zur Herstellung von Polyetheralkoholen durch Anlagerung von Alkylenoxiden an H-funktionelle Startsubstanzen, dadurch gekennzeichnet, daß die Startsubstanzen ausgewählt sind aus der Gruppe, enthaltend Alkohole mit mindestens zwei Hydroxylgruppen im Molekül und/oder Amine mit mindestens einer Aminogruppe im Molekül und einem Molekulargewicht von maximal 200 g/mol, und dass die Umsetzung in Anwesenheit von mindestens einer reduzierend wirkenden, nicht flüchtigen anorganischen Verbindung durchgeführt und/oder der Polyetheralkohol nach der Umsetzung mit den Alkylenoxiden einer Behandlung mit mindestens einer reduzierend wirkenden, nicht flüchtigen anorganischen Verbindung unterzogen wird.

Unter nicht flüchtig wird verstanden, dass die reduzierend wirkende Verbindung unter den Bedingungen, unter denen sie eingesetzt wird, also der Anlagerung der Alkylenoxide oder der Nachbehandlung der Polyetheralkohole, im Reaktionsgemisch verbleibt.

Die reduzierend wirkenden, nicht flüchtigen anorganischen Verbindungen sind vorzugsweise ausgewählt aus der Gruppe, enthaltend unterphosphorige und phosphorige Säure sowie deren anorganische Salze, anorganische Sulfite und Hydrogensulfite, wie Natriumsulfit, Kaliumsulfit oder Natriumhydrogensulfit, Boranate, wie Kaliumboranat und/oder Hydride, wie Lithium-Aluminiumhydrid.

Die Reduktionsmittel können der Startsubstanz vor der Anlagerung der Alkylenoxide und/oder dem Polyetheralkohol nach der Anlagerung der Alkylenoxide vor oder nach der Aufarbeitung zugegeben werden. Bei der Zugabe der Reduktionsmittel zur Startsubstanz vor der Anlagerung der Alkylenoxide wird die Bildung der sauren beziehungsweise der geruchsintensiven Nebenprodukte von vornherein unterbunden. Hierbei muss jedoch in jedem Falle das Reduktionsmittel zugegeben werden, was zu einer Verteuerung des Verfahrens führt.

In einer weiteren Ausführungsform des erfindungsgemäßen Verfahrens wird das Reduktionsmittel nach der Anlagerung der Alkylenoxide dem Polyetheralkohol zugegeben. Dabei kommt es zu einer Zerstörung der während der Anlagerung der Alkylen oxide gebildeten sauren beziehungsweise geruchsintensiven Verbindungen. Dieser Schritt kann generell bei allen Polyetheralkoholen durchgeführt werden. Es ist jedoch ebenfalls möglich, nur solche Chargen der Polyetheralkohole nach dem erfindungsgemäßen Verfahren zu behandeln, bei denen erhöhte Werte der genannten unerwünschten Nebenprodukte festgestellt wurden. Damit kann die Nachbehandlung der Polyetheralkohole mit den Reduktionsmitteln, die einen zusätzlichen Verfahrensschritt darstellt, auf die Einsatzfälle beschränkt werden, in denen sie zur Gewährleistung der Produktqualität notwendig ist.

Insbesondere können durch das erfinderische Verfahren Auswirkungen von Sauerstoff, beispielsweise Luftsauerstoff, auf den Polyetheralkohol zurückgedrängt und teilweise völlig rückgängig gemacht werden.

Die Reduktionsmittel können in einer Menge zwischen 10 und 10000 ppm, vorzugsweise zwischen 30 und 1000 ppm, zugesetzt werden. Die Zugabe der Reduktionsmittel kann vor der Zugabe der Alkylenoxide, bevorzugt vor dem Abstrippen des Wassers, welches aus der zugesetzten wässrigen Kalilauge und der Alkoholatbildung stammt, vorzugsweise als wässrige Lösung erfolgen. Diese Ausführungsform ist dann ist bevorzugt, wenn Nebenkomponenten, beispielsweise solche, deren Vorhandensein durch die Säurezahl ausgedrückt wird, auch bei korrekter Reaktionsführung üblicherweise zu erwarten sind. Produkte, die sensitiv auf diese Nebenkomponenten reagieren, sind vor allem solche Polyetheralkohole, die mit Acrylsäure umgesetzt und in dieser Form hauptsächlich für Lackanwendungen eingesetzt werden.

Die Zugabe der Reduktionsmittel nach der Alkylenoxiddosierung kann unmittelbar nach Beendigung der Alkylenoxidzugabe und/oder während der Nachreaktionszeit, das heißt bis etwa 4 Stunden nach Dosierende, erfolgen. Diese Ausführungsform bietet sich bei aufgetretenen Problemen während der Synthese an.

Schließlich kann das erfindungsgemäße Verfahren auch als eine Nachkorrektur des Herstellungsverfahrens eingesetzt werden, wobei nach der Neutralisation zur Abtrennung des basischen Katalysators, beispielsweise durch Salzsäure, Schwefelsäure oder Phosphorsäure, der Verfahrensschritt der Reduktion durchgeführt wird. Die in Anwesenheit der Reduktionsmittel ablaufenden Reaktionen sind in allen Ausführungsformen identisch.

Es können prinzipiell alle Polyetheralkohole nach dem erfindungsgemäßen Verfahren hergestellt werden. Bevorzugt wird das Verfahren bei Polyetheralkoholen, die für die Herstellung von Polyurethan-Weichschaumstoffen bestimmt sind, eingesetzt. Derartige Produkte haben zumeist ein Molekulargewicht von mindestens 1000 g/mol und damit längere Polyetherketten als solche, die für Anwendungen in Polyurethan-Hartschaumstoffen bestimmt sind. Damit kommt es im Verlaufe der Anlagerung der Alkylenoxide in höherem Maße zu Nebenreaktionen und somit zur Ausbildung der genannten Nebenprodukte. Außerdem werden Polyurethan-Weichschaumstoffe auch in höherem Maße für Anwendungen genutzt, bei denen die Geruchsentwicklung besonders störend ist, beispielsweise für Möbel, Matratzen oder Teile für Innenräume von Kraftfahrzeugen.

Die erfindungsgemäßen Polyetheralkohole werden, wie oben beschrieben, durch Anlagerung von Alkylenoxiden an Verbindungen mit aktiven Wasserstoffatomen hergestellt. Als Startsubstanzen für Polyetheralkohole, die zur Herstellung von Polyurethan-Weichschaumstoffen eingesetzt werden, kommen insbesondere Alkohole mit zwei oder drei Hydroxylgruppen oder Verbindungen mit mindestens einer Hydroxylgruppe und mindestens einer Aminogruppe zum Einsatz. Bevorzugt eingesetzt werden Alkohole mit zwei oder drei Hydroxlgruppen im Molekül, wie Ethylenglykol, Diethylenglykol, Propylenglykol, Dipropylenglykol, Glyzerin, Trimethylolpropan sowie beliebige Gemische aus mindestens zwei der genannten Alkohole. Weiterhin können natürliche, das heißt auf nachwachsenden Rohstoffen basierende, H-azide Öle, beispielsweise Rizinusöl, als Starter für die Herstellung von Weichschaum-Polyolen eingesetzt werden.

Als Alkylenoxide kommen insbesondere Ethylenoxid, Propylenoxid, Butylenoxid sowie beliebige Gemische aus mindestens zwei der genannten Alkylenoxide, vorzugsweise Ethylenoxid und/oder Propylenoxid, zum Einsatz. Besonders bevorzugt sind Gemische aus Ethylenoxid und Propylenoxid. Bei den Gemischen kann es sich um sogenannte Blöcke oder sogenannte statistische Gemische handeln. Bei der Anlagerung von Blöcken wird nacheinander jeweils nur ein Alkylenoxid gleichzeitig in den Reaktor dosiert, bei statistischen Gemischen wird eine Mischung der Alkylenoxide in den Reaktor dosiert. Für viele Anwendungen, insbesondere bei Kaltformschäumen, ist es vorteilhaft, am Kettenende einen reinen Ethylenoxidblock anzulagern.

Die Polyetheralkohole für die Anwendung in Polyurethan-Weichschaumstoffen haben zumeist eine Hydroxylzahl im Bereich zwischen 15 und 100 mgKOH/g und eine Funktionalität im Bereich zwischen zwei und drei.

Die Polyetheralkohole für den Einsatz in Polyurethan-Hartschaumstoffen werden ebenfalls durch Anlagerung von Alkylenoxiden an Verbindungen mit aktiven Wasserstoffatomen hergestellt. Als Startsubstanzen für Polyetheralkohole, die zur Herstellung von Polyurethan-Hartschaumstoffen geeignet sind, kommen vorzugsweise solche mit mindestens drei, vorzugsweise mindestens vier aktiven Wasserstoffatomen zum Einsatz. Insbesondere handelt es sich um Zucker, wie Sucrose oder Sorbit, aliphatische Amine wie Ethylendiamin, Diethylentriamin, Dimethylpropylamin oder deren höhere Homologe oder aromatische Amine, wie Toluylendiamin oder Diphenylmethandiamin. Diese Startsubstanzen können, insbesondere wenn sie bei Verarbeitungstemperatur fest sind, gemeinsam mit flüssigen Co-Startern mit den Alkylenoxiden umgesetzt werden. Als Co-Starter eignen sich prinzipiell alle Alkohole, die als Starter für Weichschaum-Polyetheralkohole eingesetzt werden können und oben beschrieben sind.

Die Anlagerung der Alkylenoxide an die Startsubstanz erfolgt zumeist in Anwesenheit von Katalysatoren. Zumeist werden basische Katalysatoren, insbesondere Alkali-Hydroxide, wie Kaliumhydroxid oder Cäsiumhydroxid, eingesetzt.

Zu Beginn der Umsetzung wird der basische Katalysator, der vorzugsweise in einer Menge von 0,05 bis 0,5 Gew.-%, vorzugsweise 0,1 bis 0,3 Gew.-%, bezogen auf das Gewicht des Polyetheralkohols eingesetzt wird, mit der Startsubstanz gemischt und die entstandene Mischung gegebenenfalls einer Destillation zur Entfernung von Wasser und leicht flüchtigen Bestandteilen unterworfen. Danach erfolgt die Dosierung der Alkylenoxide.

Die Umsetzung der Startsubstanz mit den Alkylenoxiden wird bei den hierfür üblichen Drücken im Bereich zwischen 0,1 und 1,0 MPa und den üblichen Temperaturen im Bereich zwischen 80 und 140 °C durchgeführt. An die Dosierung der Alkylenoxide schließt sich zumeist eine Nachreaktionsphase zum vollständigen Abreagieren der Alkylenoxide an. Nach der Anlagerung der Alkylenoxide, werden die Polyetheralkohole vom Katalysator befreit. Bei der Abtrennung der basischen Katalysatoren können auch die Reduktionsmittel und/oder deren Umsetzungsprodukte vom Polyetheralkohol abgetrennt werden.

In letzter Zeit haben Multimetallcyanidkatalysatoren, auch als DMC-Katalysatoren bezeichnet, Bedeutung gewonnen. Durch diese Katalysatoren kann die Bildung ungesättigter Nebenprodukte im Polyetheralkohol deutlich unterdrückt werden. Außerdem kann bei Einsatz von DMC-Katalysatoren die Raum-Zeit-Ausbeute bei der Anlagerung der Alkylenoxide gesteigert werden. Im Gegensatz zu den basischen Katalysatoren könne die DMC-Katalysatoren nach der Anlagerung der Alkylenoxide im Polyetheralkohol verbleiben. Die DMC-Katalysatoren werden zumeist in einer Menge von 25 bis 1000 ppm eingesetzt.

Nach der Anlagerung der Alkylenoxide und gegebenenfalls der Aufarbeitung werden dem Polyetheralkohol zumeist die üblichen Antioxidantien und/oder Stabilisatoren in den hierfür üblichen Mengen zugesetzt.

Durch das erfindungsgemäße Verfahren ist es überraschenderweise möglich, eine Bildung von unerwünschten Säuren, Aldehyden und/oder anderen geruchsintensiven Verbindungen im Polyetheralkohol bereits bei der Synthese wirksam zu unterdrücken sowie nachträglich, beispielsweise durch Eindringen von Sauerstoff, insbesondere Luftsauerstoff, gebildete Nebenprodukte zu eliminieren. Die Reduktionsmittel und deren Abbauprodukte können, sofern sie bereits vor der Aufarbeitung der Polyetheralkohole im Produkt vorlagen, bei der Aufarbeitung problemlos entfernt werden. Ansonsten ist es prinzipiell auch möglich, sie im Produkt zu belassen.

Die nach dem erfindungemäßen Verfahren hergestellten Polyetheralkohole zeichnen sich durch Geruchsfreiheit sowie durch gute Verarbeitungseigenschaften aus.

Die Erfindung soll an den nachstehenden Beispielen näher beschrieben werden.

### Beispiel 1 (Vergleich)

### Herstellung eines Polyetheralkohols unter Ausschluss von Luftsauerstoff.

In einen 50 Liter Reaktor wurden 2,27 kg Ethylenglykol und 0,24 kg 48-%ige Kalilauge vorgelegt. Das Reaktionsgemisch wurde auf 118°C aufgeheizt und dazu 47,49 kg Propylenoxid innerhalb von 9,5 h dosiert. Anschließend wurde das Produkt mit Wasser in einer Menge von 5 % der Batchgröße und 75 %-iger Phosphorsäure versetzt, bei 100°C 2 h gerührt und anschließend auf einen Wasserwert von weniger als 0,04 Gew.-% getrocknet. Daran schloss sich eine Filtration mit Tiefenfiltern an. Das Endprodukt hatte eine Hydroxylzahl von 106,5 mg KOH/g und wies eine Säurezahl von 0,021 mg KOH/g auf. Der Peroxidgehalt betrug 6,7 ppm Wasserstoffperoxid.

### Beispiel 2

### Herstellung eines Polyetheralkohols unter Anwesenheit von Luftsauerstoff mit nachträglicher Behandlung mit einem Reduktionsmittel

Es wurde die Rezeptur wie in Beispiel 1 eingesetzt. Nach der Zugabe des Propylenoxids und der Nachreaktionsphase wurde für 10 Minuten Luft in den Reaktor eingetragen. Anschließend wurde die Neutralisation durchgeführt. Aufgrund der oxidativen Schädigung hatte das Polyol nach der Aufarbeitung mit Phosphorsäure eine Säurezahl von 0,08 mg KOH/g, die Hydroxylzahl lag bei 106,3 mg KOH/g, der Peroxidgehalt betrug 25 ppm Wasserstoffperoxid.

Zu diesem Polyol wurden 6 g H₃PO₂ als 50 %ige wässrige Lösung dosiert und 4 h bei 90 °C gerührt. Anschließend wurden 11,5 g 48 %-ige Kalilauge, 0,5 % Macrosorb® und 2% Wasser bei 90 °C zugegeben, 1 h gerührt und das Macrosorb® abfiltriert. Nach der Behandlung hatte das Produkt eine Säurezahl von 0,023 mg KOH/g, der Peroxidgehalt lag bei 6,4 ppm Wasserstoffperoxid.

Durch die Reduktion mit unterphosphoriger Säure konnte also deutliche eine Verringerung der Säurezahl und des Peroxidgehalts erreicht werden.

## Patentansprüche

1. Verfahren zur Herstellung von Polyetheralkoholen durch Anlagerung von Alkylenoxiden an H-funktionelle Startsubstanzen, **dadurch gekennzeichnet, dass** die Startsubstanzen ausgewählt sind aus der Gruppe, enthaltend Alkohole mit mindestens zwei Hydroxylgruppen im Molekül und/oder Amine mit mindestens einer Aminogruppe im Molekül und einem Molekulargewicht von maximal 200 g/mol, und dass die Umsetzung in Anwesenheit von mindestens einer reduzierend wirkenden, nicht flüchtigen anorganischen Verbindung durchgeführt und/oder der Polyetheralkohol nach der Umsetzung mit den Alkylenoxiden einer Behandlung mit mindestens einer reduzierend wirkenden, nicht flüchtigen anorganischen Verbindung unterzogen wird.

2. Verfahren nach Anspruch 1, **dadurch gekennzeichnet, dass** die reduzierend wirkenden nicht flüchtigen anorganischen Verbindungen ausgewählt sind aus der Gruppe, enthaltend unterphorshorige und phosphorige Säure sowie deren anorganische Salze, anorganische Sulfite und Hydrogensulfite, Boranate, und/oder Hydrid.

3. Verfahren nach Anspruch 2, **dadurch gekennzeichnet, dass** als Sulfite Natriumsulfit, Kaliumsulfit oder Natriumhydrogensulfit eingesetzt werden.

4. Verfahren nach Anspruch 2, **dadurch gekennzeichnet, dass** als Boranat Kaliumboranat eingesetzt wird.

5. Verfahren nach Anspruch 2, **dadurch gekennzeichnet, dass** als Hydrid Lithium-Aluminiumhydrid eingesetzt wird.

6. Verfahren nach Anspruch 1, **dadurch gekennzeichnet, dass** die reduzierend wirkenden nicht flüchtigen anorganischen Verbindungen der Startsubstanz vor der Dosierung der Alkylenoxide zugesetzt werden.

7. Verfahren nach Anspruch 1, **dadurch gekennzeichnet, dass** die reduzierend wirkenden nicht flüchtigen anorganischen Verbindungen der Startsubstanz nach der Dosierung der Alkylenoxide zugesetzt werden.

8. Verfahren nach Anspruch 1, **dadurch gekennzeichnet, dass** die reduzierend wirkenden nicht flüchtigen anorganischen Verbindungen in einer Menge von 10-10000 ppm zugesetzt werden.
